# EUROPEAN PATENT APPLICATION

(11) **EP 0 872 225 A2**
(43) Date of publication of application: **21.10.1998**
(21) Application number: 98302824.2
(22) Date of filing: 09.04.1998
(51) Int. Cl.: A61F 13/04

(54) **Instrument for making foot cast**

(30) Priority: 12.04.1997 GB 9707474
(71) Applicant: Clayton, Philip Arthur, Bispham, Blackpool FY2 9NJ (GB); The Kendall Company (U.K.) Limited, Basingstoke, Hampshire RG24 8WG (GB)
(72) Inventor: Clayton, Philip Arthur, Thornton Cleveleys Lancashire FY5 3QQ (GB)
(74) Representative: Cardwell, Stuart Martin

(57) **Abstract**

A method of preparing a mould of a foot or a part thereof for use in subsequently forming a foot cast, and an apparatus for performing the method. The method utilises a moulding material comprising a sheet of thermoplastic material (1) which is mouldable at say 60°C. The mouldable material is heated to its moulding temperature and is located with respect to a carrier (11) which is apertured to receive a foot. The mouldable material spans the aperture. The mouldable material and the foot are then moved toward one another to facilitate moulding of the material around the foot to the extent required, whereupon the material is allowed to harden before the foot is removed. Conveniently the carrier is mounted in a supporting frame (33) which is slidably moveable with respect to a support surface (41). A heel former comprising a strip of flexible material (27) passes around the heel of the foot with the mouldable material interposed there between and has its ends secured with respect to the carrier and to hold the mouldable material in contact with the heel region of the foot.

## Description

The present invention relates to the preparation of a foot cast and describes a method and apparatus for use in preparing a foot cast and a moulding material for use in connection therewith.

More especially, the invention is concerned with preparing a mould of a foot or a part thereof which then serves as a cast for use, for example, in the manufacture of a functional foot orthosis.

At present the most common way of making a foot cast uses Plaster of Paris bandage which is applied to the foot and when set provides a means of making a model which is representative of the shape of that part of the foot. The model is usually a plaster cast. The use of Plaster of Paris has several disadvantages. Firstly, it is time consuming to apply and this in turn requires the patient to exhibit considerable patience whilst the cast is being made. The resulting mould is relatively fragile and of course it is not re-useable. In theory the mould is surplus to requirements once the model of the foot has been made. Therefore a moulding material which is re-useable could be advantageous.

It is an aim of the invention to overcome at least some of the above disadvantages associated with the use of Plaster of Paris.

Accordingly, a first aspect of the present invention provides a mouldable material for use in forming a foot cast according to the non-weight bearing system, characterised in that the material comprises a low temperature thermoplastic material.

The material has to be mouldable at a temperature which will not be uncomfortable when applied to the foot. A material which is thermoplastic at 60°C has been found to be quite suitable. It is preferable if the thermoplastic material has a memory effect although this is not essential. It is preferred to use a thermoplastic material in sheet form and that supplied under the registered trade mark ORFIT has been found suitable. A range of thicknesses are available but it has been found that a thickness of 1.6 mm is suitable.

Another aspect of the invention provides apparatus for use in forming a foot cast, the apparatus comprising a carrier to receive a mouldable thermoplastic material, the carrier having aperturing which is complimentary to the size of the foot to be moulded and the apparatus further comprising retention means for securing the mouldable material in a position spanning the aperturing.

More preferably the retention means comprise edge retention means for securing at least a portion of the periphery of the mouldable material. The aperturing has an outline which is larger than the foot for which the mould is being made. A generally V or U-shaped aperture has been found suitable especially one with a U-shape base and sides which diverge to provide a cut-out in the carrier.

In a preferred embodiment the carrier comprises a plate and is received in a frame member which is slidably movable with respect to a support member. In use a leg of the patient rests on the support member. A number of carrier plates are available having different aperture sizes permitting the selection of a carrier with a suitable sized aperture for the foot to be moulded.

A means is provided for securing the sheet of mouldable material with respect to the carrier plate and a particularly convenient way of doing this is to utilise a hook and loop type fastener such as that known by the registered trade mark Velcro. Strips of Velcro can be fitted to each carrier plate and complimentary strips fitted to the sheet of moulding material. This may be done using an adhesive backed strip of hook/loop fastener advantageously applied by dry heating the thermoplastic material. We have found that using two strips along opposite edges of the material suffices to secure the material to the carrier adequately for our purposes, but improved fixing arises by providing further strip along the bottom edge. In an alternative, the moulding material can be clamped with respect to a carrier frame, say between opposed clamping elements which engage opposite faces of the material, say at two opposite edge regions or around its entire periphery. Alternatively, the clamping elements may entrap the moulding material and the carrier plate.

A further advantageous feature of the invention is the provision of a heel cup former which is used to draw the mouldable material around the heel of the foot being moulded. A preferred heel cup former comprises a strip of flexible material having Velcro secured to opposite ends to permit it to be fixed and drawn tight around the heel of the user with the moulding material between the foot and the heel cup former. Conveniently strips, of Velcro material are applied to the other side of the carrier plate to receive the respective ends of the heel cup formers.

A further aspect of the invention comprises a method of forming a foot cast according to a non-weight bearing system, the method comprising the steps of heating a thermo plastic material until it is mouldable, moulding the material around the foot to form an impression thereof and allowing the material to harden, and removing the foot.

The impression of the foot can be used as a cast for example in the manufacture of functional foot orthosis.

More particularly the method comprises securing the mouldable material in a carrier and moving the carrier and foot towards one another to press the mouldable material against the foot. The mouldable material is brought into contact with the foot by moving the carrier with respect to the support so that as it moves up to and past the sole of the foot, the mouldable material is drawn around the foot and can be moulded into contact therewith. The method further comprises applying a heel cup former to the outside of the mouldable material to hold it in contact with the heel.

A preferred moulding material comprises a sheet of low temperature thermoplastic material which is heated in hot water at 60°C to render it mouldable. A preferred material is re-useable in that, by applying dry heat or by heating in hot water at 60°C, it returns to its original flat shape; ie it has a so called "memory" effect.

The present invention will now be described further by way of example only with reference to the accompanying drawings; in which:-
Figure 1 is a perspective view of part of the apparatus for use in performing the invention,
Figures 2 & 3 illustrate the front and rear views of a carrier plate for use with the apparatus of Figure 1,
Figure 4 illustrates a sheet "mouldable material" adapted for use in the invention,
Figure 5 is a perspective view of strapping for use in performing the invention, and
Figure 6 is a perspective view illustrating the apparatus in use.

Referring firstly to Figure 4 of the drawings, there is illustrated a sheet of low temperature thermo plastic material (1) which has respective strips of adhesive backed hook and loop fastener type material (3,5) secured along opposite edges (7,9). A further strip (shown in dotted outline at 8) is advatageously applied along the bottom edge. Conveniently, hook/loop type material such as that known by the registered trade mark Velcro is used. It may be secured to the mouldable material by dry heating the thermoplastic material. More preferably Velcro having a self adhesive backing is employed and it has been found that after cleaning of the mouldable material where the Velcro is to be applied with a suitable chemical cleaner (such as a neoprene thinner) to remove surface grease and/or other release agents, etc., the Velcro adheres readily. A preferred mouldable material is that made under the name ORFIT (Probably a registered Trade Mark) which is thermoplastic at 60°C. The preferred material has a memory effect which facilitates its re-use by reheating to 60°C. A sheet of material 300 mm x 150 mm x 1.6 mm thick has been found suitable for most purposes.

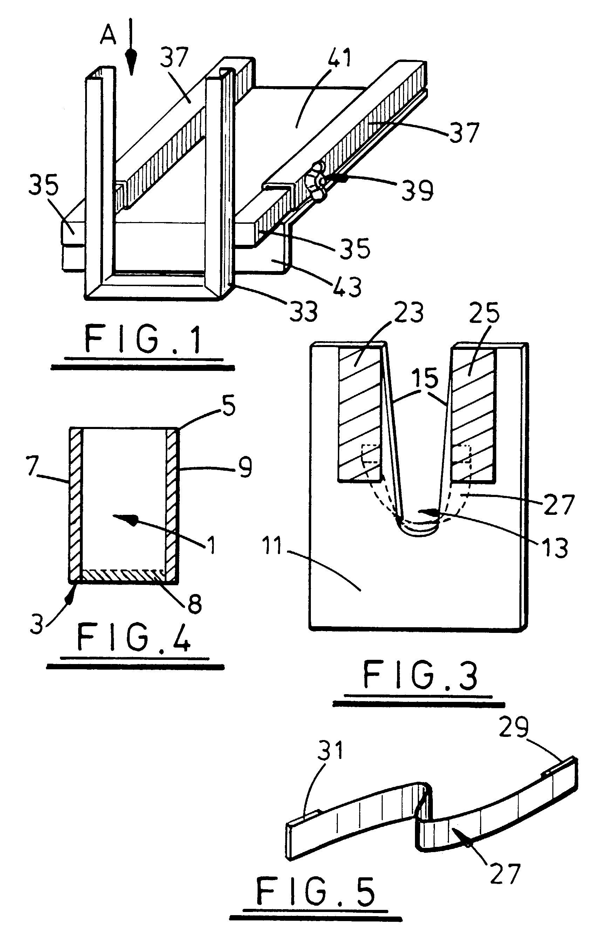

Referring to Figures 2 & 3, there is illustrated the front and rear sides respectively of a carrier plate (11). The carrier plate is preferably relatively rigid and conveniently comprises a plastics, aluminium, steel or any other convenient material. The carrier plate has an aperture (13) which is open to one edge of the plate and has side edges (15) which converge towards a base portion (17). The shape of the aperturing generally reflects the shape of a typical foot and a selection of different carrier plates are available with different size apertures to suit different size feet. In practice a number of carrier plates can be used for a number of different feet sizes. The front of the carrier plate is provided with two strips of hook/loop fastening material (19,21) to mate with the complimentary strips (7,9) on the mouldable material. A further strip 20 may be provided as shown in dotted outline to mate with strip 8 where provided. By this means the mouldable material can be secured with respect to the carrier plate spanning across the aperture (13). As illustrated in Figure 2, the strips of hook/loop material (19,21) conveniently extend from the top to the bottom of the carrier plate (11).

The rear of the carrier plate is also provided with two strips of hook/loop fastening material (23,25) extending part way along the carrier plate from the upper edge thereof. Figure 3 shows in dotted outline a heel cup former (27) which is further illustrated in Figure 5. The heel cup former comprises a strip of flexible material such as rubber, or the equivalent plastics material, of the order of 25 mm wide and 1 mm thick and the opposite ends thereof are provided with hook/loop fastening material (31,29) to mate with the complimentary strips (23,25) on the carrier plate. Elasticated Stockinette has been found suitable as an alternative. The purpose of the heel cup former will be described hereinafter.

Referring now to Figure 1 there is illustrated apparatus for receiving the carrier plate (11) comprising a U- shaped frame element (33) made up of channel section members to receive slidably in the direction of arrow (A) a carrier plate (11). The rear face of the carrier is visible when viewed in the direction of Figure 1. The U-shape frame element has limbs (35) secured thereto and these limhs are received slidably in tube elements (37). Locking nut (39) provides a means of locating the frame element with respect to the support structure (41) to which the guide tubes (37) are secured. The support structure (41) is conveniently provided with a downturned flange (43).

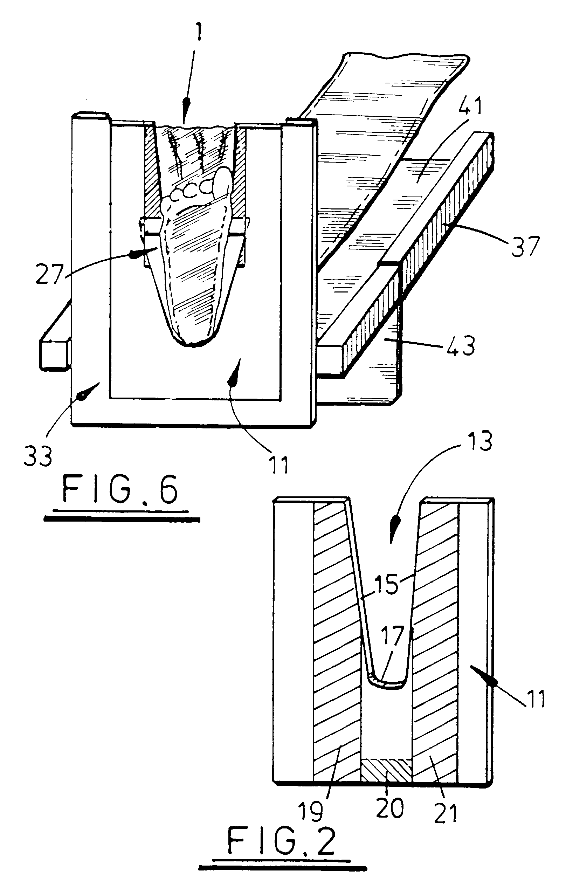

The use of the apparatus will now be described in conjunction with Figure 6. Firstly, a sheet of mouldable material having fastening means (5,7) secured along opposite edges thereof is placed in hot water at 60° until the material has softened. The material is then applied to the front face of the carrier plate (11) and the carrier plate is inserted into the frame element (33). The support structure (41) is placed at a convenient height so that the patient can rest his lower leg onto the support structure (41) with his foot projecting beyond the end of the returned flange portion (43). The frame element (33) is then moved towards the foot so that the mouldable material is pressed against the base of the foot causing the mouldable material to deform around it. The aperturing in the carrier plate allows passage of the plate beyond the sole of the foot and usually by a distance in the order of 50-75 mm at which point the frame element is secured with respect of the support structure by use of the lock nut (39). The foot and the mouldable material can then be manipulated so that an accurate impression is formed of the foot. The foot is held in place until the thermo plastic material hardens. In order to assist in the formation of an undistorted impression of the heel, the above described heel cup former is applied to the outside of the plastics material to draw it into contact with the heel of the patient. The free ends of the former are engaged with the hook/loop fastening elements (23,25) so as to hold the thermo plastic material in place whilst it hardens.

Once the mould material has hardened the foot can be removed and the mould is then available for use as a cast for example in the manufacture of a functional foot orthosis.

The use of the thermoplastic material has the advantage that it can be re-used once a cast of the foot has been produced.

It will be noted from the above description that the impression of the foot is produced in what is termed in the art a non weight bearing manner thereby reproducing the "at rest" or so called neutral position of the foot.

## Claims

1. Apparatus for use in forming a foot cast, the apparatus comprising a carrier to receive a mouldable thermoplastic material, the carrier having aperturing which is complimentary to the size of a foot to be moulded, and the apparatus further comprising retention means for securing the mouldable material in a position spanning the aperturing.

2. Apparatus as claimed in claim 1 in which the carrier comprises a plate and is received in a frame member which is slidably moveable with respect to a support member.

3. Apparatus as claimed in claim 1 or 2 in which the mouldable material comprises a sheet and is secured with respect to the carrier utilising mutually cooperable releasable hook and loop type fastening means.

4. Apparatus as claimed in anyone of the preceding claims and further comprising a heel cup comprising a strip of flexible material adapted to be secured with respect to the carrier.

5. Apparatus as claimed in anyone of the preceding claims in which the mouldable material is a low temperature thermoplastic material.

6. Apparatus as claimed in claim 5 in which the mouldable material is mouldable at 60°C.

7. A method of forming a foot cast mould according to a non-weight bearing system, the method comprising the steps of:- heating a thermoplastic material until it is mouldable; moulding the material around a foot to form an impression thereof; allowing the material to harden; and removing the foot.

8. A method as claimed in claim 7 and further comprising securing the mouldable material in a carrier across as aperture thereof; and moving the carrier and foot towards one another so that the foot passes through the aperture in the carrier to permit the mouldable material to engage the foot and to be moulded therearound.

9. A method as claimed in claim 7 or 8 and further comprising applying a heel cup former to the outside of the material to hold it in contact with the heel.

10. A mouldable material for use in forming a foot cast according to the non-weight bearing system, characterised in that the material comprises a low temperature thermoplastic material.

11. A mould for use in forming a foot cast according to the non-weight bearing system characterised in that the mould is made from a low temperature thermoplastic material.

12. A mouldable material is used in claims 10 or 11 characterised in that it is mouldable at 60°C.
